# NEUE EUROPÄISCHE PATENTSCHRIFT

(11) **EP 0 148 426 B2**
(45) Veröffentlichungstag und Bekanntmachung des Hinweises auf die Entscheidung über den Einspruch: **10.11.1993**
(45) Hinweis auf die Patenterteilung: 02.11.1989
(21) Anmeldenummer: 84114893.5
(22) Anmeldetag: 07.12.1984
(51) Int. Cl.: B65D 51/00

(54) **Verfahren zum Herstellen von pharmazeutischen Stopfen, Kolben od. dgl.**
Method to produce pharmaceutical stopper pistons or the like
Méthode de fabrication des bouchons pharmaceutiques, pistons ou similaires

(30) Priorität: 22.12.1983 DE 3346351
(43) Veröffentlichungstag der Anmeldung: 17.07.1985
(60) Teilanmeldung: 86111484.1
(73) Patentinhaber: Pharma Gummi Wimmer West GmbH, 52249 Eschweiler (DE)
(72) Erfinder: Wimmer, Hans, D-5100 Aachen (DE)
(74) Vertreter: Patent- und Rechtsanwaltssozietät, Schmitt, Maucher & Börjes

(56) Entgegenhaltungen:
- DE-A- 2 146 421
- DE-A- 3 231 179
- FR-A- 1 136 064
- GB-A- 1 061 810
- GB-A- 2 071 066
- US-A- 3 198 368
- US-A- 3 552 591
- US-A- 3 708 886
- US-A- 3 760 969

## Beschreibung

Die Erfindung betrifft ein Verfahren zur Herstellung eines im wesentlichen aus Gummi od. dgl. elastomerem Werkstoff bestehenden pharmazeutischen Stopfens, Kolbens od. dgl. Dichtung zum Verschließen oder Unterteilen einer Flasche, eines Spritzenzylinders od. dgl. Behälters, wobei der Stopfen, Kolben od. dgl. in einem dem Behälterin- nenraum zugewandten Dichtbereich eine Beschichtung aus einem fluorierten Polymerfilm od. dgl. Inertfilm aufweist, die diesen Stopfen-Dichtbereich an seiner der Wand der Behältermündung Zugewandten Seite nur teilweise umschließt, so daß ein weiterer Teil des unbeschichteten Stopfen-Dichtbereiches an der Behältermündung dichtend zur Anlage kommt.

Zum Abdichten und Verschließen von Medikamentenflaschen werden Stopfen von unterschiedlicher Form und aus unterschiedlichen Werkstoffen eingesetzt (vgl. z.B. DE-A-19 01 239, DE-A-19 46 566).

Häufig bestehen derartige Stopfen aus natürlichem odar synthetischem Gummi, gummielastischen oder reinen Thermoplaston. Gerade die elastischen Eigenschaften derartiger Werkstoffe gestatten es, daß die entsprechenden Stopfen die Toleranzen von Flaschenmündungen bzw. Spritzenzylindern ausgleichen und einen Behälter od. dgl. auch über einen längeren Zeitraum gut abdichten können.

Die in Glasflaschen, Spritzenzylindem od. dgl. Behältern verschlossen aufzubewahrenden, in der Regel flüssigen oder pulverförmigen pharmazeutischen Zubereitungen stellen jedoch sehr unterschiedliche Anforderungen an den zu verwendenden Stopfenwerkstoff. So ist beispielsweise die chemische oder biologische Verträglichkeit eines Gummistopfens mit dem Flascheninhalt gefordert. Außerdem ist bei sauerstoff- oder feuchtigkeitsempfindlichen Flascheninhalten die Gas- oder Wasserdampfdichtigkeit des Verschlußstopfens wesentlich. Weiterhin darf der Stopfenwerkstoff keine Veränderung des therapeutischen Wertes des Behälterinhaltes verursachen, z.B. dadurch, daß er schädliche oder ein pharmazeutisches Präparat verändernde Inhaltsstoffe abgibt bzw. Bestandteile aus der pharmazeutischen Zubereitung od. dgl. aufnimmt. Zahlreiche Werkstoffzusammensetzungen für Stopfen können die vorstehend erwähnten Probleme nur teilweise lösen.

Man hat daher bereits ein stopfenförmiges Verschlußelement geschaffen, das mit einem ringförmigen Steg dichtend in den Flaschenhals eines Behälters eingreift (DE-A-32 31 179). Dieses vorbekannte Verschlußelement weist oberhalb seines Steges eine tellerförmige und nach außen angeordnete Verschlußkappe auf, die auf der Flaschenmündung aufliegt. Um den Behälter gut abzudichten, ist die Verschlußkappe des vorbekannten Verschlußelementes mit einem eine Umbördelung der Flaschenmündung hintergreifenden Metallverschluß oder Deckel fest auf der Flaschenmündung angebracht.

Der mit dem Behälterinhalt in Berührung kommende ringförmige Steg ist von einer aus einem inerten Fluorokohlenstoff-Harz bestehenden Schicht umgeben, die auf diesen Bereich des Verschlußelementes beispielsweise durch Vulkanisieren aufgebracht ist. Dabei ist diese inerte Schicht so bemessen, daß sie auch die Umbördelung der Flaschenmündung noch überdeckt.

Derartige Schichten aus chemisch sehr inerten und widerstandsfähigen Polymeren können das Problem der Unverträglichkeit zwischen Stopfenwerkstoff einerseits und Behälterinhalt andererseits zwar weitgehend beseitigen; der zu ihrer Herstellung erforderliche, hochwertige Folienwerkstoff ist jedoch sehr teuer, was sich insbesondere bei solchen Verschlußelementen nachteilig auswirkt, deren untere, die Flaschenmündung beaufschlagende und mit dem Behälterinhalt in Berührung kommende Seite nahezu vollständig mit einer derartigen inerten Schicht versehen ist.

Vor allem aber haben die die inerte Schicht beim Stopfen bildenden Folien eine erhebliche Härte und sind wenig verformbar, so daß es zwischen dem Glasbehälter od. dgl. einerseits und dem mit einer fluorierten Polymerfolie versehenen Stopfen andererseits nicht zu der erwünschten sicheren Abdichtung kommt, wenn die Polymerfolie den gesamten in die Behältermündung, in einen Spritzampullen-Zylinderoderdgl. einzusetzenden Stopfenhals ummantelt. Der relativ harte und wenig elastische, das Glas beaufschlagende Polymerfilm vermag zwar gegen Flüssigkeiten abzudichten, nicht jedoch gegen das Eindringen von Gasen und Bakterien. Die Gas- und Wasserdampfdurchlässigkeit an der Grenzfläche zwischen der fluorierten Folie des Stopfenhalses einerseits und der Innenwand des Behälters andererseits kann so hoch werden, das z.B. ein Vakuum innerhalb eines Behälters nicht über ausreichend lange Zeiträume aufrechterhalten werden kann. So weist beispielsweise das bereits oben beschriebene, vorbekannte stopfenartige Verschlußelement einen im äußeren Randbereich der die Flaschenmündung beaufschlagenden Seite derVerschlußkappe angeordneten, von der inerten Schicht freigehaltenen konzentrischen Vorsprung auf, der einen im Inneren evakuierten Behälter beispielsweise gegen ein Eindringen von Umgebungsluft zumindest für die Zeit zwischen der Verkapselung und dem darauf folgenden Verschluß mittels des Metallverschlusses oder Deckels abdichtet. Ein solches Vakuum ist z.B. bei zahlreichen gefriergetrockneten Produkten erforderlich.

Durch das Aufeinandertreffen zweier harter Stoffe, wie beispielsweise des fluorierten Polymerfilmes auf Glas, ist die bei medizinischen Behältern notwendige Sterilität nicht über längere Zeit zu gewährleisten. Wenn, wie beispielsweise die DE-A-2146421 und die DE-A-32 31 179 zeigen, nur ein schmaler Abschnitt der flanschartigen Verschlußkappe eines gummielastischen Verschlußelementes eine Behälterstimseite abdeckt, ist eine ausreichende Abdichtung gegen Gas-und Wasserdampfdurchlässigkeit nicht mehr mit der notwendigen Sicherheit gegeben.

Auch lassen sich bei der Herstellung der vorerwähnten, mit einer fluorierten Polymerfolie versehenen Stopfen, insbesondere, wenn bei diesen Folien größere Verformungen vorgenommen werden müssen, Faltenbildungen und Poren regelmäßig kaum vermeiden. Die Faltenbildung führt dann zu neuen Problemen bzgl. der Dichtigkeit zwischen Glas und Verschlußstopfen; die Poren ermöglichen einen unerwünschten Kontakt zwischen dem Behälterinhalt und dem Gummistopfen.

Man hat bereits auch einen Stopfen aus gummielastischem Material geschaffen, über dessen in das Behälterinnere ragenden Dichtbereich eine formangepaßte Schutzkappe aus widerstandsfähigem Material aufgesteckt und dort mechanisch gehalten ist; dabei ist diese Schutzkappe so bemessen, daß ein weiterer Teil des unbeschichteten Dichtbereiches des Stopfens an der Behältermündung dichtend zur Anlage kommt (GB-A-1 061 810). Die Kappe und dergummielastische Stopfenteil sind dort also nicht untrennbar durch Vulkanisieren verbunden.

Durch das nachträgliche mechanische Aufbringen gestaltet sich aber nicht nur das Herstellungsverfahren bei diesem vorbekannten Stopfen aufwendiger, vielmehr bringt dies auch die Gefahr mit sich, daß die Schutzkappe in den Behälter gesogen wird, wenn dieser unter Vakuum - wie beispielsweise bei gefriergetrockneten Produkten - steht. Um den vorbekannten Stopfen mit der Schutzkappe zusammensetzen zu können, muß dieser eine gewisse Mindestwandstärke aufweisen, was wiederum bei inerten, regelmäßig spröden Werkstoffen zu schlechten Abdichtverhältnissen führt. Auch läßt sich durch das mechanische Zusammensetzen von elastomerem und inertem Stopfenteil insbesondere an den Verbindungsfugen während des Gebrauchs des Stopfens kaum die beispielsweise bei medizinischen Präparaten oft notwendige Sterilität einhalten.

Ein Aufvulkanisieren des gummielastischen Stopfenmaterials auf einen Inertfilm kann beim Herstellungsprozeß jedoch dazu führen, das gummielastischer Werkstoff am Außenrand des kappenartigen Inertfilms vorbeifließt, ihn an der später dem Behälterinnenraum zugewandten Seite benetzt bzw. verunreinigt und seine ihm ursprünglich zugedachte Funktion, nämlich die chemische Neutralität des Stopfens, wieder zunichte macht.

Aus all dem geht hervor, daß bereits seit langer Zeit nach einem Verfahren und einem damit herstellbaren Stopfen od. dgl. Dichtung gesucht wird, in dem die vorteilhaften Werkstoffeigenschaften von Elastomeren, deren elastisches Verhalten und gute Abdichtung auch am unebenen Bereich etwa von Glasbehälter-Mündungen einerseits und die vorteilhaften Eigenschaften von inerten Kunststoffen, nämlich deren chemische Neutralität andererseits, gleichzeitig miteinanderkombiniertwerden können, ohne daß die jeweiligen Nachteile solcher Werkstoffe ins Gewicht fallend in Kauf genommen werden müssen.

Es besteht daher die Aufgabe, ein kostengünstiges Verfahren zur Herstellung von Stopfen, Kolben od. dgl. Dichtungen zu schaffen, die eine gute Dichtigkeit gegen Flüssigkeit, Gase und Wasserdampf sowie gegen das Eindringen von Keimen usw. aufweisen. Dabei soll der chemisch inerte Bestandteil des Stopfens, Kolbens od. dgl. Dichtung in dem Verfahren so aufgebracht werden, daß eine Wechselwirkung zwischen dem Stopfenwerkstoff od. dgl. einerseits und dem Behälterinhalt andererseits beispielsweise durch nachträgliche Verschmutzungen oder ein unbeabsichtigtes Lösen des inerten Materials zuverlässig vermieden wird.

Die erfindungsgemäße Lösung besteht bei dem Verfahren der eingangs erwähnten Art darin, daß die Herstellung des Stopfens od. dgl. in mehreren Arbeitsschritten erfolgt, wobei im ersten Schritt zunächst ein unvulkanisiertes Gummifell zusammen mit einem fluorierten Polymerfilm od. dgl. Inertfilm in eine Kaliberplatte eines ersten Formwerkzeuges eingedrückt wird und dabei entsprechende Abschnitte des Polymerfilms od. dgl. und des Gummifelles die der (den) in der Kaliberplatte vorgesehenen Hohlform(en) entsprechende Umrißform annehmen, wobei sich der an der Wand der Hohlform anliegende Polymerfilm od. dgl. unter Einwirkung von Hitze untrennbar mit dem innenliegenden Teil des Gummifelles verbindet und zu je einem inneren Dichtteil des Stopfens vulkanisiert, daß im nächsten Arbeitsschritt die so geformten, mit dem fluorierten Polymer od. dgl. beschichteten inneren Dichtteile des Stopfens aus dem ersten Formwerkzeug entnommen und im nächsten Arbeitsschritt so aus dem beschichteten Gummifell ausgestanzt werden, daß der Oberrand jedes inneren Gummi-Dichtteiles radial etwas mit einem Durchmesser über den durch die Hohlform vorgegebenen Durchmesser vorsteht, daß dieser überstehende Oberrand im nächsten Arbeitsschritt, bei dem das innere Dichtteil in die Hohlform(en) eines zweiten Formwerkzeuges eingebracht werden, diese Hohlform(en) nach oben derart abdichtet, daß im nächsten Arbeitsschritt beim Zusammenfügen des inneren Dichtteils mit einem Stopfenaußenteil ein Eintreten von unvulkanisiertem Gummimaterial des späteren Stopfenaußenteils in dem Bereich zwischen fluoriertem Polymerfilm od dgl. und der Wand der Hohlform des zweiten Werkzeuges verhindert wird.

Durch das erfindungsgemäße Verfahren ist es nicht nur möglich, inertes Folienmaterial auch lediglich auf einem Teilbereich des beispielsweise in einem Flaschenhals ragenden Dichtbereiches eines Stopfens unlösbar aufzubringen, sondern auch weitgehend zu vermeiden, daß den Stopfen oder dgl. in einem weiteren Arbeitsschritt vervollständigendes, zunächst noch nicht ausvulkanisiertes elastisches Stopfenmaterial sich in den Bereich des inerten Folienmaterials hinein bewegt und es dann zu einer unerwünschten Wechselwirkung zwischen dem elastischen Stopfenmaterial einerseits mit dem Behälterinhalt andererseits kommen kann. Dementsprechend erhält man einen Stopfen oder dgl., bei dem der Behälterinhalt praktisch nur mit dem fluorierten Polymerfilm oder dgl. chemisch inerten Filmteil des Stopfens in Verbindung kommen kann, während ein weiterer Teil des Stopfenhalses oder dgl. mit seinem gummmielastischem Werkstoff an der Innenwand des Behälters für eine gute Abdichtung gegen Flüssigkeiten, Gase, Wasserdampf sowie für die Abdichtung gegen das Eindringen von Keimen durch entsprechenden unmittelbaren Kontakt von Gummistopfen und Behälter Glas oder dgl. sorgt. Im Bereich des Stopfenhalses oder dgl. werden die Vorteile von bisher bekannten Verschlußwerkstoffen miteinander vereinigt, ohne daß deren jeweilige Nachteil in Kauf genommen werden müssen. Dabei erstreckt sich der vom Werkstoff her teuere Polymerfilm oder dgl. nur über einen verhältnismäßig kleinen Teil des gesamten Stopfens, der deshalb vergleichsweise preiswert herstellbar ist. Zudem kann in dem erfindunggemäßen Verfahren beispielsweise auch ein dreischichtiger Stopfenaufbau realisiert werden, wobei der stets dünne, materialsparende Überzug einen geometrisch im wesentlichen genau begrenzten Bereich des Stopfens überdeckt. Dabei werden hier unter « Schicht oder « Werkstoffschicht » insbesondere in Achsrichtung benachbart zueinander angeordnete Stopfenabschnitte verstanden, beispielsweise das inerte Folienmaterial als « erste Schicht » der damit unlösbarverbundene elastomere Werkstoff als « zweite Schicht und der in einem letzten erfindungsgemäßen Verfahrensschritt auf den vorgenannten elastomeren Werkstoff aufzubringende weitere elastomere Werkstoff, der das äußere Teil des Stopfens bildet, als « dritte Schicht ». In diesem Sinne ist auch ein « dreischichtiger » Stopfenaufbau zu verstehen, insbesondere wenn, wie noch genauer ausgeführt wird, die außen liegende elastomere Werks toffschicht eine andere Zusammensetzung hat als die elastomere Werkstoffschicht, die unlösbar mit der inerten Folienschicht in Verbindung steht. Der in dem erfindungsgemäßen Verfahren hergestellte Stopfen oder dgl. vermag sich aufgrund seiner inerten Folienschicht gegenüber dem Behälterinhalt weitgehend neutral zu verhalten, hat darüber hinaus aber auch den besonderen Vorteil, daß durch die unlösbare Verbindung dieser Schicht mit dem elastomeren Stopfenmaterial im Nahtbereich dieser Schichten sich keine Verunreinigungen festsetzen können und daher die geforderte Sterilität des Stopfens oder dgl. auf einfache Weise eingehalten werden kann. Bei dem erfindungsgemäßen Verfahren wird das innere, mit einem chemisch inerten Film zu versehende Stopfenteil in separaten Produktionsschritten gefertigt. Dabei wird das Gummifell, auf das z. B. zuvor der chemisch inerte Film auflamiert wurde, als zunächst ebener Verbund oder auch einzeln in die Herstellungsform gebracht und dort mittels Druck und Wärme in die vorgesehene Form verformt und dabei gleichzeitig untrennbar verbunden. Bei diesem Verformungsvorgang wirkt das Gummimaterial als eine Art elastisches Druckpolster welches dazu dient, die Folie bei den in der Form herrschenden Temperatur- und Druckbedingungen in die gewünschte Endform zu bringen. Dadurch wird eine Falten- und Porenbildung beim Tiefziehen der Folie weitgehend vermieden.

Zusätzliche Weiterbildungen des erfindungsgemäßen Verfahrens sind in den Verfahrens-Unteransprüchen und in der Beschreibung aufgeführt. Dabei befaßt sich das Verfahren nach Anspruch 4 mit einem besonders schonend ausgestalteten Tiefziehvorgang. Da bei dem erfindungsgemäßen Verfahren die inerte Folie mit dem elastomeren Werkstoff nicht mechanisch verbunden wird, können auch sehr dünne Materialschichten beispielsweise des vergleichsweise teueren fluorierten Polymerfilmes od. dgl. verwendet werden. In Anspruch 6 ist ein Verfahren zur Herstellung eines Stopfens od. dgl. Dichtung beschrieben, der sich besonders gut beispielsweise im Hals einer Flasche festlegen kann. Anspruch 7 behandelt einen Stopfen od. dgl. Dichtung, durch dessen dreischichtigen Aufbau die Werkstoffeigenschaften der einzelnen Stopfenbereiche noch weiter optimiert werden können.

Weitere vorteilhafte Weiterbildungen des Herstellungsverfahrens für pharmazeutische Stopfen, Kolben oder dgl. Dichtungen sind in den Unteransprüchen 8 sind 9 aufgeführt.

Nachstehend wird die Erfindung anhand vorteilhafter Ausführungsbeispiele in Verbindung mit den Figuren noch näher erläutert.

Es zeigt:
Fig. 1 einen Längsschnitt durch eine mit einem Stopfen verschlossene Medikamentenflasche,
Fig. 2 eine teilweise im Schnitt gehaltene Seitenansicht eines Stopfens ähnlich dem aus Fig. 1,
Fig. 3 einen Teillängsschnitt einer offenen Herstellungsform für die Stopfen,
Fig. 4 einen Teilschnitt der geschlossenen Herstellungsform entsprechend Fig. 3,
Fig. 5a und 5b die aus einem ersten « Gummifell ausgestanzten Stopfen-Innenteile,
Fig. 6 die zwaite Herstellungsform füreinen Stopfen in geöffneter Stellung und im Teillängsschnitt,
Fig. 7 einen Teillängsschnitt einer End-Fertigungsform ähnlich Fig. 6,
Fig. 8 einen Teillängsschnitt durch einen gem.
Fig. 7 hergestellten Stopfen, der sich innerhalb einer Flaschenmündung befindet,

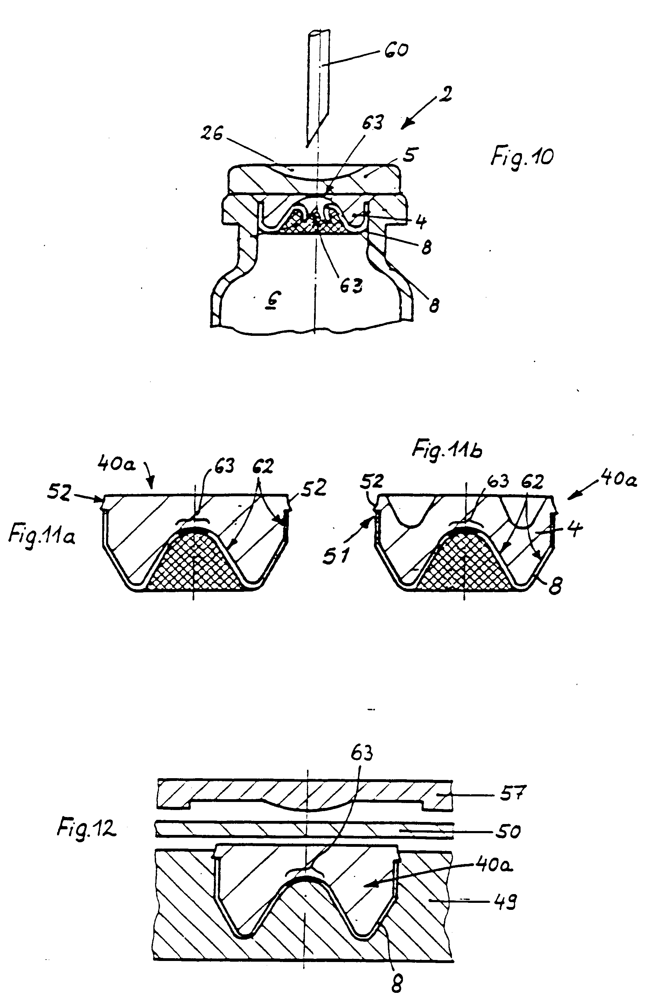

Fig. 9 einen Längsschnitt durch einen Stopfen, der sich im Oberteil einer Medikamentenflasche befindet und durch den ein Infusionsdorn gesteckt ist,
Fig. 10 einen Längsschnitt durch einen Stopfen entsprechend Fig. 9, aus dem der Infusionsdorn herausgezogen ist,

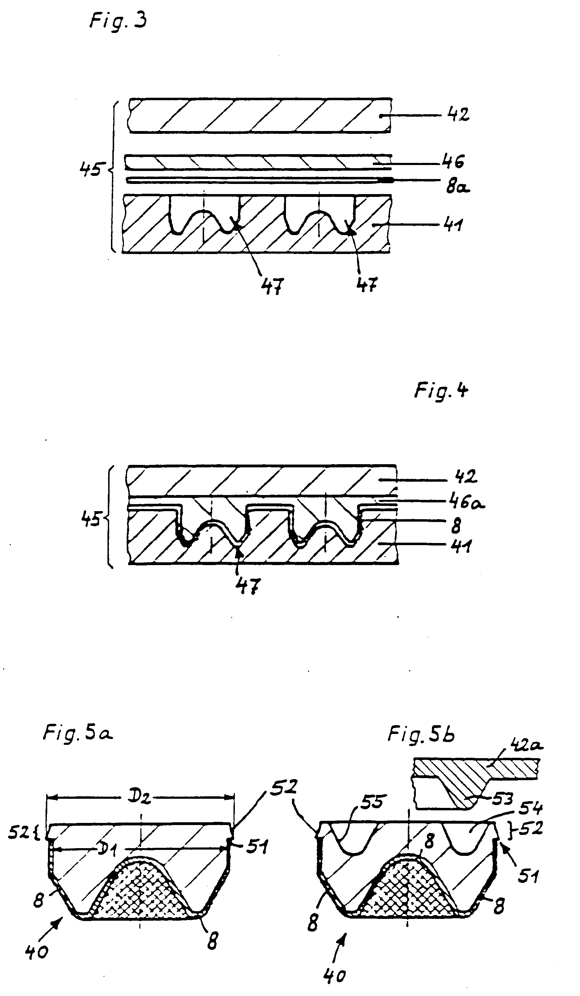

Fig. 11a und 11 b Stopfenunterteile ähnlich Fig. 5a und 5b, bei denen die Verbindungsfläche zwischen dem fluorierten Polymerfilm und dem gummielastischen Stopfenteil im Durchstichbereich für einen Infusionsdorn unterbrochen ist,
Fig. 12 einen Teillängsschnitt einer offenen Herstellungsform für das Stopfenunterteil gem. Fig. 11a,

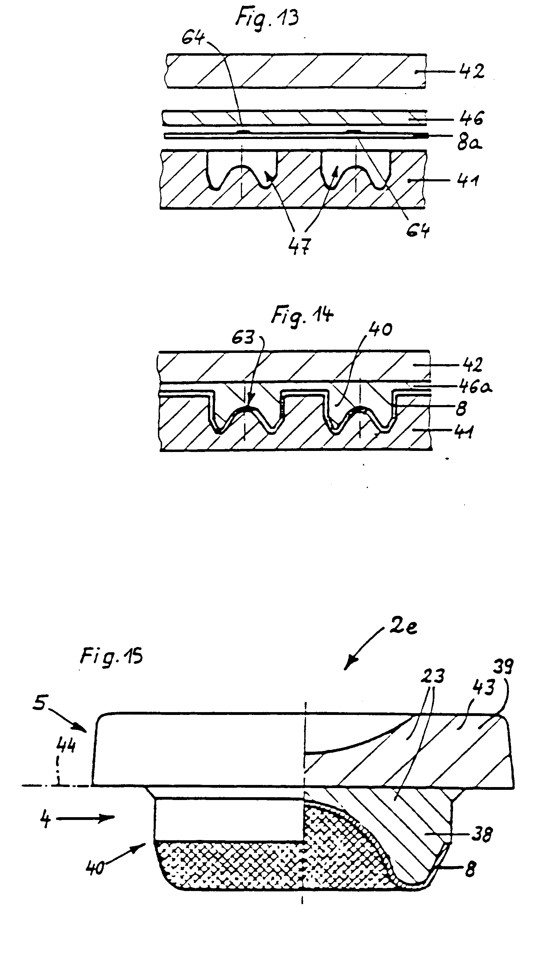

Fig. 13 einen Teillängsschnitt einer offenen Herstellungsform für die Stopfen ähnlich Fig. 3, jedoch für eine etwas abgewandelte Herstellungsweise,
Fig. 14 einen Teillängsschnitt der Herstellungsform gemäß Fig. 13 im geschlossenen Zustand,

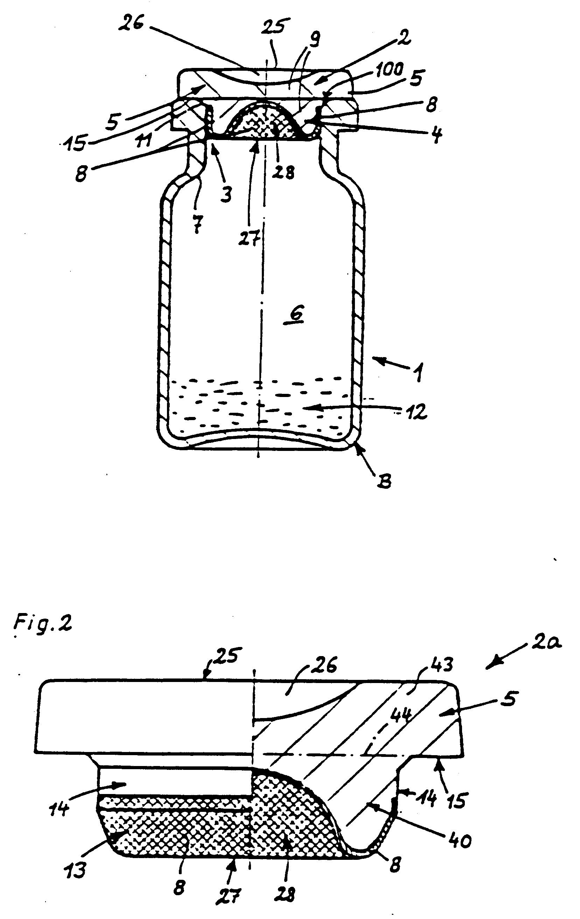

Fig. 15 eine teilweise im Schnitt dargestellte Seitenansicht eines Stopfens ähnlich dem aus Fig. 2, dessen gummielastisches Teil aus zwei unterschiedlichen Werkstoffen besteht,

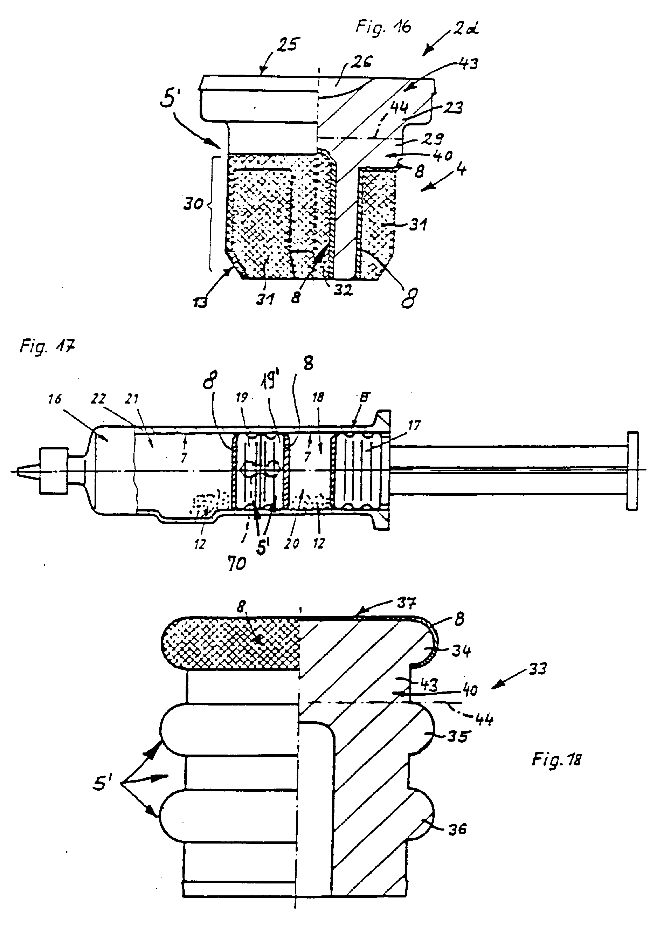

Fig. 16 eine teilweise im Schnitt dargestellte Seitenansicht eines Gefriertrockenstopfens,
Fig. 17 eine teilweise im Schnitt gehaltene Seitenansicht einer Zweikammer-Spritzampulle, in der sich ein Kolben als Zylinderabschluß und ein weiterer Kolben als Unterteilung des Injektionsspritzen-Zylinders befinden und,
Fig. 18 eine teilweise im Schnitt dargestellte Seitenansicht eines Kolbens für eine Spritzampulle.

Fig. 1 zeigt eine Medikamentenflasche 1, die durch einen im ganzen mit 2 bezeichneten pharmazeutischen Stopfen verschlossen ist, der nachstehend auch kurz mit Stopfen 2 » bezeichnet ist. Dieser ist als Formstopfen ausgebildet und weist in bekannter Weise einen in die Flaschenmündung 3 eingesetzten Stopfenhals 4 sowie einen diesen flanschartig radial überragenden Stopfenaußenteil auf. Der Stopfen 2 ist an seinem dem Behälterinnenraum 6 zugewandten Stopfenbereich teilweise mit einem fluorierten Polymerfilm oder dgl. überzogen und durch Vulkanisieren untrennbar damit verbunden ist, wobei jedoch nur ein Abschnitt (vgl. z.B. Abschnitt 30 in Fig. 16 und Abschnitt 34 in Fig. 18) des Stopfenschaftes oder dgl. von dem fluorierten Polymerfilm 8 oder dgl. abgedeckt ist und ein weiterer Teil 14; 29 (vgl. Fig. 2 bzw. Fig. 16) des im wesentlichen zylindrischen Dichtungsabschnittes sowie ein dem Behälterinnenraum 6 ; 20, 21 abgewandtes Stopfenteil 5 bzw. 5' eine beschichtungsfreie Dichtfläche aufweist. Dabei wird hier unter der « Behälterwand » nicht nur die Innenwand 7 des Behälterinnenraumes der Medikamentenflasche 1 gemäß Fig. 1, sondern dort speziell die den Stopfenhals 4 aufnehmenoe Wand 100 der Flaschenmündung und z. B. bei der Ausführung gemäß Fig. 17 die Innenwand des Injektionszylinders 22 der Spritzampulle 16 verstanden.

Bezüglich der abgewandelten Ausführungsformen des Stopfens 2 wird insbesondere auf die nachstehende Beschreibung der Figuren 16 bis 18 verwiesen.

Als fluorierte Polymerfilme od. dgl. kommen vorzugsweise folgende fluorierte Hochpolymere in Frage:
Polytetrafluorethylen (PTFE),
Tetrafluorethylenperfluoropropylen-Copoly mer (FEP),
Perfluoralkoxy-Copolymer (PFA),
Ethylen-Tetrafluorethylen-Copolymer (ETFE),
Polyvinylidenfluorid (PVDF),
Polyvinylfluorid (PVF).

Obgleich dies die bevorzugten Werkstoffe für den fluorierten Polymerfilm 8 sind, kann dieser auch aus anderen Werkstoffen bestehen, die einerseits gegenüber dem Behälterinhalt 12 inert und biologisch verträglich, andererseits, gegebenenfalls unter Zwischenschaltung von Haftvermittlern, mit dem ihnen benachbarten Bereich des gummielastischen Teils des Stopfens 2 genügend fest verankerbar sind. Solche Werkstoffe werden in der Anmeldung auch kurz « Inertfilm » genannt. Der gummielastische Teil 9 des Stopfens 2 kann im wesentlichen aus natürlichem oder synthetischem Gummi, gummielastischen oder reinen Thermoplasten bestehen. Diese Werkstoffe werden hier kurz mit « Gummi », die daraus bestehenden Stopfen kurz mit « Gummistopfen » und deren aus diesen Werkstoffen bestehende Teile mit « gummielastische Teile bezeichnet.

Fig. 2 zeigt einen dem Stopfen gem. Fig. 1 ähnlichen Stopfen 2a. Man erkennt gut, daß bei diesem Formstopfen 2a der fluorierte Polymerfilm 8 bis etwas oberhalb eines konischen Einführteiles 13 des Stopfenhalses 40 reicht. Die Abdichtung der Medikamentenflasche 1 erfolgt durch das Einpressen des zylindrischen Teiles 14 des Stopfenhalses 40 in die Flaschenmündung 3. Diese insbesondere gegen das Eindringen von Gas, Wasserdampf, Bakterien usw. dienende Abdichtung kann noch dadurch vergrößert werden, daß die Unterseite 15 des flanschartig vorstehenden Stopfenoberteiles 5 auf die Stirnseite 10 der Flaschenmündung 3 aufgepreßt wird, was beispielsweise in der Praxis gewöhnlich mit entsprechenden Bördelkappen erfolgt.

Fig. 1 und 2 zeigen gut, daß der Behälterihalt 12 auch unter ungünstigen Umständen praktisch nicht in Reaktion mit dem gummielastischen Teil des Stopfens 2a treten kann, dieser Teil jedoch eine Abdichtfunktion des Behälterinnnenraumes 6 gegenüber der Umgebung übernimmt, welche der vergleichsweise harte, fluorisierte Polymerfilm 8 nicht gegenüber der gewöhnlich mit Unebenheiten versehenen Behälterinnenwand 7 übernehmen kann.

Aus Fig. 1 und 2 wird auch deutlich, daß der Inertfilm 8 einen geometrisch mehr oder weniger exakt begrenzten dünnen Überzug auf dem gummielastischen Teil 9 bildet. Dies wird durch das erfindungsgemäße Verfahren ermöglicht, indem beispielsweise die in Fig. 1 und 2 gezeigten Stopfen kostengünstig hergestellt werden können, die die vorteilhaften Werkstoffeigenschaften von Elastomeren, deren elastisches Verhalten und gute Abdichtung auch an unebenen Auflageflächen einerseits und die vorteilhaften Eigenschaften von inerten Kunststoffen, nämlich deren chemische Neutralität, andererseits gleichzeitig miteinander verbinden, ohne daß die jeweiligen Nachteile solcher Werkstoffe ins Gewicht fallend in Kauf genommen werden müssen.

Das erfindungsgemäße Herstellungsverfahren läuft folgendermaßen ab :

Für dan zuerst herzustellenden Stopfenteil 40 (vgl. Fig. 2) ist eine erste Formplatte 41 (vgl. Fig. 3) vorgesehen, die mit einer ersten Gegenplatte 42 das erste Formwerkzeug 45 für den ersten Arbeitsgang bildet. Dieses ist in einem Teillängsschnitt in geöffneter Stellung in Fig. 3 schematisiert dargestellt. Das zuerst herzustellende Stopfenteil ist in Fig. 2 mit 40 bezeichnet und, stark schematisiert, zu dem ergänzend herzustellenden zweiten Stopfenteil 43 durch eine strichpunktierte Linie 44 abgetrennt angedeutet. Diese strichpunktierte Linie 44 kann auch mit der Trennebene zwischen den unterschiedlichen Stopfenwerkstoffen 38 und 39 zusammenfallen (vgl. Fig. 15).

Bei der Ausführung gem. Fig. 2 ist die Trennebene im Hinblick auf eine geeignete Herstellungsmöglichkeit unter Berücksichtigung der Napfform der Polymerfolie 8 gewählt.

Zwischen die geöffneten Teile 41, 42 des im ganzen mit 45 bezeichneten ersten Werkzeuges sind, stark schematisiert, ein unvulkanisiertes « Gummifell 46 », und ein unverformter, flacher Polymerfilm 8a angeordnet. Unter « Gummifell » versteht man in der einschlägigen Fachwelt eine Gummischicht von einer gewissen Längen- und Breitenausdehnung, mit dem gewöhnlich eine Form an den Rändern so weit überdeckt werden kann, daß ein Arbeitsgang möglich ist. Der zunächst noch ebene folienartige fluorierte Polymerfilm 8a gem. Fig. 3 kann sich bei diesem Herstellungsschritt bereits in fester Verbindung mit dem Gummifell 46 befinden oder er kann, wie Fig. 3 schematisiert zeigt, unabhängig und mit einem Abstand vom Gummifell 46 zwischen die Formteile 41 und 42 eingebracht werden.

Die Verbindung des fluorierten Polymerfilmes 8a auf das unvulkanisierte Gummifell 46 hat unter anderem den Vorteil des einfacheren Einbringens der Teile 46, 8a zwischen der ersten Formplatte 41 und der ersten Gegenplatte 42. Das getrennte Einbringen von Gummifell 46 und ebenem, fluoriertem Polymerfilm 8a (Fig.3) hat unter anderem den Vorteil, daß ein besonderer Arbeitsgang zum Verbinden von Gummifell 46 und fluoriertem Polymerfilm 8a eingespart werden kann.

Fig. 4 zeigt das in Fig. 3 dargestellte erste Formwerkzeug in der Schließposition. Hier ist das zunächst noch unvulkanisierte, jetzt mit 46a bezeichnete Gummifell in die dem zuerst herzustellenden Stopfenteil 40 entsprechenden Hohlformen 47 eingedrückt worden. Dabei ist derfluorierte, zunächst ebene Polymerfilm 8a in die gewünschte napfartige Form gebracht worden, wobei er gem. Fig. 4 mit « 8 » bezeichnet ist. Wird das erste Formwerkzeug 45 (Fig. 4) unter Druck geschlossen, wird der unvulkanisierte Gummiteil-Werkstoff zusammen mit dem fluorierten Polymerfilm 8a in die Hohlform 47 (= Kaliberform 47) eingepreßt. Dadurch wird der fluorierte Polymerfilm 8 od. dgl. tiefgezogen, wie gut aus Fig. 4 erkennbar ist Es ist ein wichtiger Gesichtspunkt der Erfindung, daß das zugehörige Gummimaterial des Gummifells 46 bzw. 46a als elastisches Druckpolster auf diesen fluorierten Polymerfilm 8 od. dgl. wirkt.

Dabei kann zweckmäßigerweise die erste Formplatte 41 bareits eine erhöhte Temperatur haben (ca. 120 °C bis 210 °C, vorzugsweise 150 °C bis 190°C) und man kann den Druck, der sich in den Kaliberformen 47 einstellt, langsam ansteigen lassen. Der Tiefziehvorgang des fluorierten Polymerfilmes aus einer ebenen Form 8a gem. Fig. 3 in die napfförmige Form 8 gemäß Fig. 4 kann dementsprechend schonend durchgeführt werden, wobei auch der Werkstoff des Gummifells als elastisches Druckpolster günstig wirkt.

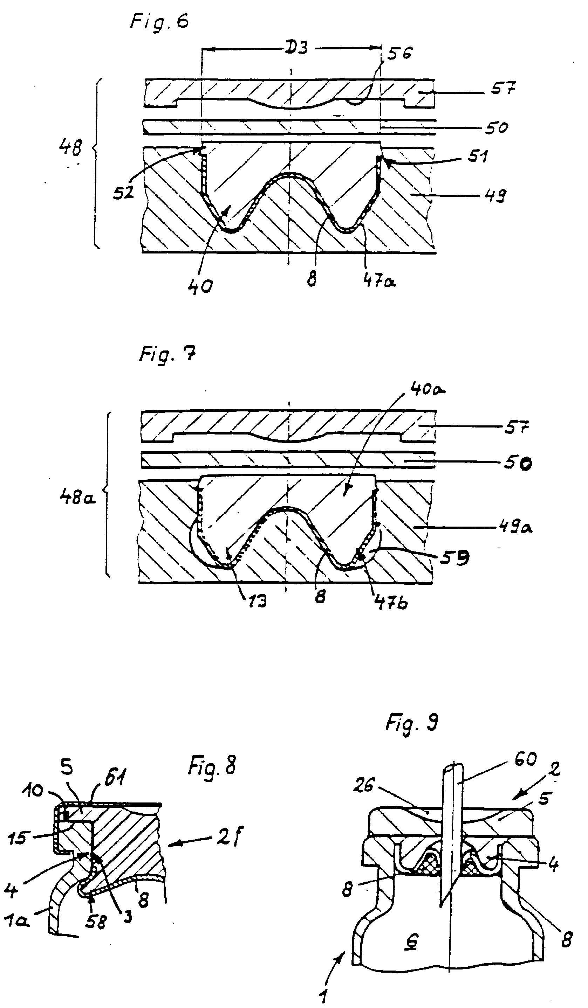

Beim und/oder nach dem Schließen des ersten Formwerkzeuges wird das unvulkanisierte Gummifell 43 bzw. 46a durch Temperatureinwirkung anvulkanisiert. Je nach Vorbehandlung des fluorierten Polymerfilmes 8a, 8 od. dgl. wird dieser dabei physikalisch oder chemisch mit dem gummielastischen Teil des späteren Stopfens untrennbar verbunden, in der Regel durch Vulkanisieren auf der gesamten gemeinsamen Verbindungsfläche. Nach Beendigung dieses Vorganges wird das anvulkanisierte Gummifell 46a, auf dessen einer Seite sich der fluorierte Polymerfilm 8 befindet, aus dem ersten Formwerkzeug 45 entfernt und die auf diese Weise zuerst hergestellten Stopfenteile 40 werden ausgestanzt und zwar derart, daß beim Einlegen der zuerst hergestellten Stopfenteile 40 in das zweite Formwerkzeug 48 (vgl. Fig. 6) das zuerst hergestellte Stopfenteil 40 eine Abdichtung dagegen gewährleistet, daß in die zugehörigen Kaliberformen 47a der Unterkaliberplatten 49 des zweiten Formwerkzeuges ein Vorbeifließen des Werkstoffes vom zweiten Gummifell 50 (Fig. 6) am fluorpolymer-beschichteten, bereits fertigen Stopfenteil 40 vermieden wird.

Dies wird dadurch erreicht, daß beim Einstecken dieses zuerst gefertigten Stopfenteiles 40 in die Unterkaliberplatte 49 des zweiten Formwerkzeuges mittels dieses Oberrandes 52 ebenfalls eine Abdichtung erfolgt; beispielsweise dadurch, daß im Bereich dieses Oberrandes 52 der Durchmesser D3 der hohlen Kaliberform 47a etwas kleiner als der Durchmesser D2 des Oberrandes 52 ist. Dadurch ergibt sich im Bereich dieses Oberrandes 52 eine Abdichtung durch den lippenförmigen Oberrand 52 des zuerst hergestellten Stopfenteiles 40, wie gut in Fig. 5a und 6 erkennbar.

Diese Abdichtung kann aber auch durch eine entsprechende Formgebung des (nicht gezeichneten) Schnittstempels im zweiten Arbeitsgang (Ausstanzen der zuerst gefertigten Stopfenteile 40) erreicht werden, so daß das erste Gummifell 46a zuerst etwas zusammengedrückt wird, ehe der Ausstanzvorgang erfolgt. Dieser wird dann bei einem entsprechend großen Durchmesser durchgeführt. Nach dem Stanzen dehnt sich der entsprechende Teil des zuerst hergestellten Stopfenteiles 40 etwas radial nach außen, so daß der Stanzrand des zuerst gefertigten Stopfenteiles 40 einen etwas größeren Außendurchmesser D2 als der Außendurchmesser der napfartigen inerten Polymerfolie 8 aufweist.

Wenn das zuerst herzustellende Stopfenteil 40 eine größere axiale Erstreckung erhalten bzw. die zugehörige Inertfolie 8 besonders tiefgezogen werden soll, kann die erste Gegenplatte 42a des ersten Formwerkzeuges eine Kalibererhebung 53 haben, durch die eine entsprechende Aussparung 54 an deroberen Stirnseite des zuerst gefertigten Stopfenteiles 40 entsteht (vgl. Fig. 5b). Mit einer solchen Ausbildung kann man auch die Verbindungsfläche 55 des zuerst hergestellten Stopfenteiles 40 mit dem danach herzustellenden zweiten Stopfenteil 43 vergrößern, wie gut aus Fig. 5b erkennbar ist.

Als weiterer Arbeitsschritt wird dann das zweite Formwerkzeug 48 (Fig. 6) geschlossen. Der Werkstoff des zweiten Gummifelles 50 fließt in die Höhlung 56 des Oberkalibers 57 des zweiten Formwerkzeuges und es entsteht ein Stopfen, wie er ähnlich in Zusammenhang mit Fig. 1 und 2 näher beschrieben wurde.

Fig. 7 zeigt noch einen gegenüber dem Arbeitsschritt nach Fig. 6 abgewandelten Arbeitsschritt. Dort ist die Unterkaliberplatte 49a eines zweiten Formwerkzeuges 48a gegenüber der Ausführung nach Fig. in folgender Weise abgeändert: Die hohle Kaliberform 47b gem. Fig. 7 weist eine Hohlwulst 59 auf, die den konischen Einführungsteil 13 des dortigen zuerst hergestellten Stopfenteiles 40a radial überschreitet. Dieses zuerst gefertigte Stopfenteil 40a einschließlich seines napfförmigen inerten Polymerfilmes 8 od. dgl. ist noch nicht endgültig ausgeformt. Beim Schließen der Form 48a entsteht nicht nur der im letzten Herstellungsabschnitt anzufertigende Stopfenteil43, sondern im Unterkaliber49a baut sich genügend Druck auf, um das bereits vorgeformte Stopfenteil 40a zusammen mit der inerten Polymerfolie 8 od. dgl. ein weiteres Mal zu verformen. Man erhält dann einen Stopfen 2f (Fig. 8), der an seinem inneren Ende eine umlaufende, etwas radial vorstehende Wulst 58 entsprechend der Form der Hohlwulst 59 im Unterkaliber 49a hat. Ein solcher Stopfen 2f kann sich z. B. besonders gut im Hals der Flasche 1a festlegen.

Obgleich das bevorzugte Anwendungsgebiet der erfindungsgemäß hergestellten Stopfen 2 Verschlüsse für Medikamentenflaschen od. dgl. Behälter mit pharmazeutischen Zubereitungen ist, sind diese Stopfen 2 und deren Herstellungsverfahren auch gut im Bereich von anderen hochempfindlichen Behälterinhalten anzuwenden.

Das sich erfindungsgemäß in mehrere Arbeitsschritte unterteilende Herstellungsverfahren wie auch der Stopfen 2 haben unter anderem folgende Vorteile : Da der Stopfenhals 4 im Durchmesser kleiner als ein diesen flanschartig radial überragender Stopfenaußenteil 5 ist und stets nur ein Teil des Stopfenhalses 4 mitfluoriertem Polymerfilm 8 od. dgl. ummantelt ist, kommt man mit einer vergleichsweise kleinen Menge von teuerem Polymerfilm 8 od. dgl. aus. Dies fällt vorteilhaft beim Herstellungsverfahren in mehreren Arbeitsschritten ins Gewicht : Im ersten Herstellungsschritt mit dem ersten Formwerkzeug 45 können die Kaliberformen 47 für die zuerst herzustellenden Stopfenteiie 40 bzw. entsprechende « Formnester enger beieinander angeordnet werden, als wenn gleich der gesamte Stopfen 2 mit entsprechend großem Stopfenaußenteil 5 herzustellen wäre. Dementsprechend können in einem solchen Formwerkzeug 45 pro Flächeneinheit wesentlich mehr Teile 40 pro Pressung gefertigt werden. Das ermöglicht eine erhebliche Ersparnis an hochwertigem, sehrteuerem Folienwerkstoff aus fluoriertem Polymer- oder anderen chemisch inerten Filmen 8a. Unter Umständen kann die Ersparnis bis zu 50 % dessen gehen, was bei einteiliger Herstellung von Stopfen notwendig wäre, wenn nahezu deren gesamte Unterseite beschichtet würde.

Die fluorierten Polymerfilme 8a, wie sie z. B. in Fig. 3 dargestellt sind, können extrudiert, gegossen oder geschält sein. Ihre Dicken liegen zweckmäßigerweise zwischen 0,01 und 0,5 mm.

Wie bereits z. T. vorstehend erwähnt, bestehen wesentliche Vorteile des erfindungsgemäßen Stopfens 2 oder dgl. Dichtung im folgendem : Ein Teil der dem empfindlichen Behälterinhalt zugewandten Stopfenoberfläche ist mit einem fluorierten Polymerfilm 8 oder dgl. Inertfilm nicht nur umgeben sondem unmittelbar und unlösbar damit verbunden. Zwischen dem fluorierten Polymerfilm 8 oder dgl. und dem damit unlösbar verbundenen elastomeren Werkstoffanteil des Stopfens besteht praktisch keine wenn auch noch so schmale Fuge, in die Verschmutzungen oder dgl. hinein gelangen könnten. Der fluorierte Polymerfilm 8 oder dgl. kann gegenüber der Behälterinnenwand 7 bzw. 100 eine Dichtfunktion hinsichtlich des Behälterinhaltes 12 derart übernehmen, daß dieser praktisch nicht in unerwünschter Weise mit dem restlichen, gummielastischen Teil, z. B. Teil 23 des Stopfens 2d (Fig. 16) oder dgl. reagiert. Dies ist nicht nur bei einfach geformten, z. B. zylindrischen oder Formstopfen (z. B. Fig. 1 u. 2) sondern auch bei sehr zerklüfteten Stopfen wie Gefriertrockenstopfen (vgl. Fig. 16) möglich. Auch kann der Bereich, in dem ein fluorierter Polymerfilm 8 oder dgl. Inertfilm den Stopfen 2 oder dgl. umfaßt, den unterschiedlichen Bedürfnissen angepaßt werden (vgl. Fig. 16 bis 18).

Nach Beendigung des zuletzt beschriebenen Arbeitsschrittes wird der Stopfen 2 oder dgl. aus seinem Gummifell 50 ausgestanzt. Ein Wesentlicher Gesichtspunkt für das erfindungsgemäße Vertahren besteht darin, daß eine fluorierte Polymerfolie 8a oder dgl. aus inertem Werkstoff bestehende Folie in sehr günstiger, sicherer und materialsparenderweise tiefgezogen werden kann, wobei auch noch der Werkstoff für den gummielastischen Teil 23 des Stopfens 2 oder dgl. gewisser- maßen ein elastisches Polster beim Tiefziehen des Polymerfilms 8 bildet. Vorteilhaft ist auch, daß das Tiefziehen der fluorierten Polymerfolie 8 oder dgl. im gleichen Arbeitsgang wie das Herstellen des zuerst anzufertigenden Stopfenteiles 40 erfolgt. Außerdem kann beim gleichzeitigen Tiefziehen der Polymerfolie 8 oder dgl. mit der Herstellung des zuerst antzufertigenden Stopfenteiles 40 das unerwünschte Schrumpfen von Polymerfolien vermieden werden, welches in der Regel auftritt, wenn solche Polymerfolien alleine tiefgezogen werden. Durch das erfindungsgemäße Verfahren wird weitgehend sichergestellt, daß dieser Inertfilm ein geometrisch genügend exakt begrenzten dünnen Überzug auf dem gummielastischen Stopfenmaterial bildet.

Das Vorbereiten einer Polymerfolie 8 oder dgl. zum Erreichen einer mechanischen Verbindung gegenüber dem gummielastischen Teil 23 des Stopfens 2 oder dgl. erfolgt gewöhnlich auf chemische Weise, wobei die entsprechende Oberfläche der Potymerfolie 8 genügend « griffig » gemacht wird, so daß sich der Werkstoff des gummielastischen Teiles 23 - im Mikrobereich gesehen - auf mechanische Weise durch Verhaken, Hintergreifen usw. beim Vulkanisieren fest mit der Polymerfolie 8 verbinden kann.

In Fig. 8 ist noch eine mit 61 bezeichnete, bekannte Bördelkappe zu erkennen, mit der man ein genügend festes Aufpressen der Unterseite 15 des Stopfenoberteiles 5 auf die Stirnseite 10 der Behältermündung 3 erreicht.

Stopfen, wie sie vorstehend beschrieben und beispielsweise in den Figuren 1 u. 2 dargestellt wurden, haben sich in Versuchen gut bewährt, wenn sie nur einmal durchstochen werden.

Es hat sich jedoch herausgestellt, daß solche an sich sehr vorteilhaften Stopfen bei bestimmten Anwendungen Nachteile haben können, wenn sie beispielsweise zweimal durchstochen werden müssen. In Fig. 9 ist beispielsweise ein Stopfen 2 ähnlich dem in Fig. 1 gezeigten Stopfen dargestellt, dessen Stopfenhals 4 in einem dem Flascheninneren 6 zugewandten Abschnitt mit einem Polymerfilm 8 ummantelt ist. Führt man in einen solchen Stopfen 2 einen Infusionsdorn 60 ein, verformt dieser den Polymerfilm 8 an der Durchstichstelle. Unter Bildung eines Durchstich-Schlitzes verbiegt sich der Polymerfilm 8 od. dgl. in Richtung des Behälterinneren 6. Entfernt man den Infusionsdorn 60, wie in Fig. 10 erkennbar, so behält der fluorierte Polymerfilm 8 od. dgl. in etwa die nach dem Durchstich des Infusionsdornes 60 eingenommene Form bei.

In Fig. 10 ist dabei auch der im Polymerfilm 8 entstandene Schlitz 63 bei der früheren Durchstichstelle des Infusionsdorns 60 gut erkennbar. Die Polymerschicht 8, die auch im Bereich der Durchstichstelle fest mit dem gummielastischen Werkstoff des Stopfenhalses 4 in Verbindung steht, kann dann diese gummielastische Durchstichstelle 63 zumindest bereichsweise, im wesentlichen im Nachbarbereich des Polymerfilms 8, etwas offen halten. Das hängt damit zusammen, daß der fluorierte Polymerfilm 8 od. dgl. durch den vorbeschriebenen Herstellungsprozeß stark verdehnt worden ist und das Bestreben hat, in seine ursprüngliche Form zurückzukehren (Memory-Effect). Wenn nun nach dem ersten Durchstechen des Stopfens 2 mit einem Infusionsdorn 60 und nach dessen Entfernen mit dem Präparat z. B. Mischbewegungen durchgeführt werden oder das Präparat Gasdruck beim Lösen entwickelt, kann sich in solchen Fallen das Verhalten des fluorierten Polymerfilmes 8 od. dgl. ungünstig auswirken, indem der schlitzartig aufgeplatzte und verformte Polymerfilm 8 od. dgl. die Gummi-Durchstichstelle 63 etwas offen hält. Dadurch kann gegebenenfalls Flüssigkeit bzw. Medikament-Anteil beim Schütteln oder durch Überdruck durch die Durchstichstelle 63 nach außen austreten. Dies kann unter Umständen in mehrfacher Hinsicht nachteilig sein. Z. B. ist die Dosierung des Medikamentes nicht mehr sicher. Auch kann der Flascheninhalt chemisch unerwünschte Auswirkungen auf die handhabende Person haben.

Um die zuletzt beschriebenen, in speziellen Fällen auftretenden Nachteile zu vermeiden, wird die Erfindung wie folgt weitergebildet: Die Verbindungsfläche 62 (Fig. 11a, 11 b), mit welcher der fluorierte Polymerfilm 8 eine Haftverbindung mit dem Stopfenhals 4 eingeht, wird im Bereich der Durchstichstelle 63 für den Infusionsdorn 60 antweder gar nicht haftfähig gemacht oder, was in der Praxis vorzugsweise erfolgt, haftunfähig gemacht. Der vorzugsweise aus PTFE bestehende Inertfilm 8 wird nämlich normalerweise durch Ätzen seiner späteren Verbindungsfläche 62 erst haftfähig gemacht, da die Ätzung eine dafür geeignete Oberflächenstruktur ermöglicht. Im Ausführungsbeispiel gemäß Fig. 11a, 11 wird die durch Ätzen hervorgerufene Flächenstruktur, vorzugsweise mittels Erhitzen der Durchstichstelle 63 des Polymerfilmes 8 aufgehoben. Dann verbindet sich die PTFE-Folie 8 in diesem Bereich nicht mit dem gummielastischen Werkstoff des Stopfenhalses 4. Die besondere Ausbildung des Inertfilms 8 an der Durchstichstelle 63 ist in Fig. 11 und 12 durch die ausgezogene schwarze Linie im Bereich 63 angedeutet. Die Herstellung eines ersten Stopfenteiles 40 mit einer unterbrochenen Verbindungsfläche an der Durchstichstelle 63 erfolgt, wie in Fig. 12 angedeutet, im übrigen ähnlich wie vorbeschrieben, insbesondere in Verbindung mit Fig. 6.

Eine solche gewünschte Unterbrechung der Verbindungsfläche 62 an der Durchstichstelle 63 kann nicht nur, wie vorbeschrieben, dadurch erreicht werden, daß die durch Ätzung hervorgerufene Oberflächenstruktur des Inertfilms 8 mittels Hitze wieder aufgehoben wird. Der bandförmig oberhalb der Formplatte 41 (Fig. 13) befindliche fluorierte Polymerfilm 8a kann beispielsweise auch durch chemische Zugaben an der späteren Durchstichstelle 63 so behandelt werden, daß dort bei der Herstellung des ersten Stopfenteiles 40 keine Verbindung zwischen dem gummielastischen Werkstoff des Stopfen halses 4 einerseits und der Verbindungsfläche 62 des Polymerfilms 8a andererseits auftritt.

In Fig. 13 sind dazu z. B. räumlich genau bemessene Portionen 64 einer Chemikalie aufgebracht, welche beim Herstellungsvorgang eine dort unerwünschte Haftfähigkeit des Inertfilms 8 bzw. 8a verhindert. Im übrigen entspricht der Herstellungsvorgang gem. Fig. 13 und 14 im wesentlichen dem bereits in Verbindung mit den Figuren 3, 4, 6 und 7 beschriebenen und vorstehend näher erläuterten Herstellungsvorgang.

Bei einem Stopfen 2, dessen Unterteil 40a (Fig. 11a, 11 b) gem. dem in Verbindung mit den Figuren 9 bis 14 beschriebenen Verfahren hergestellt ist, verformt sich der fluorierte Polymerfilm 8 od. dgl. Inertfilm beim Eindringen des Infusionsdorns 60 in dieses Stopfenteil 40 a in gleicher Weise, wie in Verbindung mit Fig. 9 und 10 beschrieben wurde. Dies hängt auch damit zusammen, daß der gewöhnlich aus PTFE bestehende Inertfilm sich bei Temperaturen, die Gummi oder gummiartiger Werkstoff noch verträgt, nicht bleibend verformt. Dementsprechend ist beim Herstellungsprozeß gemäß der Erfindung das Problem vorhanden, daß PTFE od. dgl. in Verbindung mit dem Gummi unter der Formplatte 41 verformt wird, aber entsprechende Spannungen in Richtung einer Rückbildung zur alten Form des Inertfilmes 8a zurückbleiben. Beim Herausziehen des Infusionsdorns 60 gem.

Fig. 10 verbleiben im Durchstechbereich 63 Teile des Polymerfilms 8 auch in der verformten Lage, wie schematisch in Fig. 10 angedeutet. Wenn jedoch an dieser Durchstichstelle 63 keine Verbindung zwischen dem Polymerfilm 8 od. dgl. einerseits und dem gummiartigen Werkstoff des Stopfens 2 andererseits besteht, kann sich der Stopfenhals 4 in der üblichen Weise nach dem Entfernen des Infusionsdornes 60 wieder schliessen. Man kann den Flascheninhalt dann unbedenklich schütteln, wie das beispielsweise zur Rekonstitution von gefriergetrockneten Produkten zweckmäßig ist.

Unter « Rekonstitution » wird hier verstanden, daß ein solches Produkt durch Zugabe von Flüssigkeit wieder aufgelöst wird. Auch wenn sich im Flascheninneren 6 bei Zugabe einer Flüssigkeit oder eines Medikamentenanteils und/oder beim Schütteln ein Überdruck ergibt, erhält man noch einen ausreichend sicheren Verschluß der Medikamentenflasche 1.

Durch die verschiedenen Arbeitsschritte können mit Hilfe des erfindungsgemäßen Verfahrens auch mehrschichtige, beispielsweise dreischichtige Stopfen hergestellt werden, die durch ihren dreischichtigen Aufbau die Werkstoffeigenschaften der einzelnen Stopfenbereiche noch weiter optimieren.

So zeigt Fig. 15 ein abgewandeltes Ausführungsbeispiel der bisher beschriebenen Stopfen : Dort besteht beim gummielastischen Teil 23 des Stopfens 2e der Stopfenhals 4 aus einem anderen gummielastischen Werkstoff als das Stopfenaußenteil 5, was durch die unterschiedliche Schraffur angedeutet ist. Dadurch ist es möglich, die gewünschten Werkstoffeigenschaften der beiden unterschiedlichen gummielastischen Teile 38 und 39 zu optimieren. Beispielsweise kann man beim Werkstoff des der fluorierten Polymerfolie 8 benachbarten Teiles Gummimaterial verwenden, welches besonders gut eine Verbindung mit dem Polymerfilm 8 eingeht, während man den Gummiwerkstoff, der später das Stopfenaußenteil 5 bildet, beispielsweise nach dem Gesichtspunkt guter Dichtfähigkeit, z. B. nach dem Entfernen eines Infusionsdornes 60, oder Verbindbarkeit mit dem Stopfenunterteil oder auch nach Preis-Gesichtspunkten auswählen kann.

Fig. 16 zeigt eine weitere Abwandlung eines Formstopfens 2d, der eine bei Gefriertrocknungsstopfen übliche Umrißform hat. Hier hat der Stopfenhals 4 ein äußeres, zylindrisches Dichtstück 29. In Richtung des Behälterinnenraumes 6 schließt sich daran ein inneres Ende 30 an. In diesem sind randoffene, im Querschnitt gewöhnlich kreissektorförmige Aussparungen 31 und ein zentrales Sackloch 32 ausgebildet. Das gesamte innere Ende 30 dieses Gefriertrockenstopfens 2d ist mit einem durchgehenden fluorierten Polymerfilm 8 beschichtet. Dieser Polymerfilm 8 reicht bis in den unteren Ansatzbereich des zylindrischen Dichtstückes 29, so daß der Behälterinhalt 12 vor dem direkten Kontakt mit dem gummielastischen Teil 23 dieses Stopfens 2d geschützt ist. Trotzdem ergibt sich nach dem vollständigen Einpressen des Stopfens 2d in die Behältermündung 3 eine sichere Abdichtung des zylindrischen Dichtungsstückes 29 mittels dessen aus gummielastischem Werkstoff bestehenden Abschnittes.

Das erfindungsgemäße Verfahren kann nicht nur zur Herstellung der bereits oben beschriebenen Stopfen, sondern beispielsweise auch zur Herstellung von Verschlußelementen, Kolben od. dgl. Dichtungen vorteilhaft eingesetzt werden, wie dies aus Fig. 17 deutlich wird.

Fig. 17 zeigt als abgewandeltes Anwendungsbeispiel eine im ganzen mit 16 bezeichnete Zweikammerspritzampulle. Sie ist an ihrem rückwärtigen Ende mit einem Kolbenstopfen 17 verschlossen und ihrZylinderinnenraum 18 ist von einem Trennkolben 19 in zwei Abschnitte 20 und 21 unterteilt. Die Medikamentenflasche 1 aus Fig. 2 und der Spritzenzylinder 22 werden hier auch gemeinsam als « Behälter » bezeichnet.

Auch in den Abschnitten 20 und/oder 21 des Spritzenzylinders 22 können sich entsprechend empfindliche pharmazeutische Zubereitungen befinden. Der Kolbenstopfen 17 und/oder der Trennkolben 19 können dann ähnlich wie der bereits beschriebene Stopfen 2, 2a ausgebildet sein und haben an einer oder auch an beiden ihrer Stirnflächen einen fluorierten Polymerfilm 37 (vgl. (Fig. 18).

In Fig. 17 sind die Kolbenteile 19, 19' separat hergestellt und mit einem Kupplungsstück 70 miteinander verbunden. Die beiden Inertfilme 8 liegen dementsprechend außen.

Fig. 18 zeigt einen Kolben 33 ähnlich dem Trennkolben 19 bzw. dem Kolbenstopfen 17, wie er bereits in Fig. 17 beschrieben wurde. Dieser besitzt drei radial vorstehende Dichtwülste 34, 35, 36 od. dgl. Dichtlippen. Die einer pharmazeutischen Zubereitung am nächsten stehende Dichtwulst 34 und die entsprechende Stirnseite 37 dieses Kolbens 33 sind mit einem fluorierten Polymerfilm 8 umkleidet der gemäß dem erfindungsgemäßen Verfahren unlösbar mit dem gummielastischen Werkstoff des Kolbens 33 verbunden ist.

Mit dem zylindrischen oder zumindest im wesentlichen zylindrischen Stopfen hals 4 des Stopfens 2 od. dgl. gem. Fig. 1 bis 18 kann man innerhalb einer zylindrischen oder wenigstens im wesentlichen zylindrischen Behältermündung, also ohne die in ihrer Dichtwirkung unter Umständen problematische Stirnseite eines Mündungsflansches eines Behälters 1 in Anspruch zu nehmen, eine Abdichtung des Behälterinneren 6 mit einem Stopfenhals 4 od. dgl. erreichen, bei dem zwei unterschiedliche Werkstoffe ihre jeweils vorteilhaften Werkstoffeigenschaften zur Wirkung bringen, ohne daß diese durch herstellungsbedingte Verschmutzungen oder ein unbeabsichtigtes Lösen einer Stopfenschicht wieder zunichte gemacht werden könnten: Der fluorierte Polymerfilm 8 od. dgl. Inertfilm trennt den Flascheninhalt, insbesondere während dessen Lagerzeit, gegenüber dem gummielastischen Teil des Stopfens 2. Dieser gummielastische Teil des Stopfenhalses 4 sorgt demgegenüber für eine sichere Abdichtung im Bereich des zumindest im wesentlichen zylindrischen Stopfenhalses 4 und übernimmt dabei Dichtungsfunktionen, die der fluorierte Polymerfilm od. dgl. nicht übernehmen kann.

In den Figuren 16 bis 18 sind noch diejenigen Stopfenteile, die dem jeweiligen Behälterinnenraum, z. B. den Ampullenräumen 20 u. 21 der Spritzampulle 16 abgewandt sind, mit 5' bezeichnet.

## Patentansprüche

1. Verfahren zur Herstellung eines im wesentlichen aus Gummi od. dgl. elastomerem Werkstoff bestehenden pharmazeutischen Stopfens, Kolbens od. dgl. Dichtung (2-2f) zum Verschließen oder Unterteilen einer Flasche (1), eines Spritzenzylinders (22) od. dgl. Behälters, wobei der Stopfen, Kolben od. dgl. in einem dem Behälterinnenraum (6) zugewandten Dichtbereich eine Beschichtung aus einem fluorierten Polymerfilm (8) od. dgl. Inertfilm aufweist, die diesen Stopfen-Dichtbereich an seiner der Wand der Behältermündung zugewandten Seite nur teilweise umschließt, so daß ein weiterer Teil des unbeschichteten StopfenDichtbereiches an der Behältermündung (3) dichtend zur Anlage kommt, dadurch gekennzeichnet, daß die Herstellung des Stopfens od. dgl. in mehreren Arbeitsschritten erfolgt, wobei im ersten Schritt zunächst ein unvulkanisiertes Gummifell (46) zusammen mit einem fluorierten Polymerfilm od. dgl. Inertfilm (8) in eine Kaliberplatte (41) eines ersten Formwerkzeuges (45) eingedrückt wird und dabei entsprechende Abschnitte des Polymerfilms od. dgl. (8) und des Gummifelles (46) die der (den) in der Kaliberplatte (41) vorgesehenen Hohlform(en) (47) entsprechende Umrißform annehmen, wobei sich der an der Wand der Hohlform (47) anliegende Polymerfilm od. dgl. (8) unter Einwirkung von Hitze untrennbar mit dem innenliegenden Teil des Gummifelles (46) verbindet und zu je einem inneren Dichtteil (46a) des Stopfens (2) vulkanisiert, daß im nächsten Arbeitsschritt die so geformten, mit dem fluorierten Polymer od. dgl. (8) beschichteten inneren Dichtteile (46a) des Stopfens (2) aus dem ersten Formwerkzeug (45) entnommen und im nächsten Arbeitsschritt so aus dem beschichteten Gummifell (46) ausgestanzt werden, daß der Oberrand (52) jedes inneren Gummi-Dichtteils (46a) radial etwas mit einem Durchmesser (D2) über den durch die Hohlform (47) vorgegebenen Durchmesser (D1) vorsteht, daß dieser überstehende Oberrand (52) im nächsten Arbeitsschritt, bei dem das innere Dichtteil (46a) in die Hohlform(en) (47a) eines zweiten Formwerkzeuges (48, 48a) eingebracht werden, diese Hohlform (en) nach oben (bei 51) derart abdichtet, daß im nächsten Arbeitsschritt beim Zusammenfügen des inneren Dichtteils (46a) mit einem Stopfenaußenteil (5) ein Eintreten von unvulkanisierten Gummimaterial des späteren Stopfenaußenteils in den Bereich zwischen fluoriertem Polymerfilm od. dgl. (8) und der Wand der Hohlform (47a, 47b) des zweiten Werkzeuges (48, 48a) verhindert wird.

2. Verfahren nach Anspruch 1, dadurch gekennzeichnet, daß ein unvulkanisiertes Gummifell od. dgl. Kautschukplatte mit einem fluorierten Polymerfilm od. dgl. (8), gegebenenfalls unter Verwendung von Haftmitteln, verbunden wird, zweckmäßigerweise durch Aufwalzen.

3. Verfahren nach Anspruch 1 oder 2, dadurch gekennzeichnet, daß der fluorierte Polymerfilm od. dgl. (8) und das unvulkanisierte Gummifell (46) od. dgl. Kautschukplatte miteinander verbunden werden, bevor sie zwischen die Gegenplatte (42) und die Formplatte (41) des ersten Formwerkzeuges (45) gebracht werden.

4. Verfahren nach einem der Ansprüche 1 bis 3, dadurch gekennzeichnet, daß der Schließvorgang des ersten Formwerkzeuges (45) beim ersten Arbeitsschritt so gesteuert wird, daß das unvulkanisierte Gummifell (46) od. dgl. Kautschukplatte bei langsam ansteigendem Druck in die Hohlform (en) (47) des ersten Formwerkzeuges (45) eindringt und dabei die entsprechenden Abschnitte des unvulkanisierten Gummifelles (46) od. dgl., als Druckpolster wirkend, den fluorierten Polymerfilm od. dgl. (8) riß- und faltenfrei tiefzieht.

5. Verfahren nach einem der Ansprüche 1 bis 4, dadurch gekennzeichnet, daß der zur Beschichtung des Stopfens (2) od. dgl. dienende fluorierte Polymerfilm od. dgl. (8) eine Dicke zwischen 0,01 und 0,5 Millimeter aufweist.

6. Verfahren nach einem der Ansprüche 1 bis 5, dadurch gekennzeichnet, daß das beim ersten Arbeitsschritt gefertigte innere Dichtteil (46a) des Stopfens od. dgl. (2) zunächst noch verformbar bleibt und innerhalb des zweiten Formwerkzeuges (48) eine weitere Verformung erfährt, wobei ein vorzugsweise etwas radial über den übrigen Durchmesser (D1) des Dichtteils hinausragender Wulst (58) geformt wird.

7. Verfahren nach einem der Ansprüche 1 bis 6, dadurch gekennzeichnet, daß für das Stopfenaußenteil (5) ein anderes Gummimaterial verwendet wird, als das den fluorierten Polymerfilm od. dgl. (8) tragende innere Dichtteil (46a) des Stopfes od. dgl. (2-2f).

8. Verfahren nach einem der Ansprüche 1 bis 7, dadurch gekennzeichnet, daß die Haftung des fluorierten Polymerfilms od. dgl. (8) gegenüber dem inneren Dichtteil (46a) od. dgl. durch Inertisierung in vorgegebenen Bereichen - z. B. der Durchstichstelle (63) -- vor dem Verbinden von Polymerfilm od. dgl. und innerem Dichtteil od. dgl. aufgehoben wird.

9. Verfahren nach Anspruch 8, dadurch gekennzeichnet, daß die Inertisierung durch punktuelles Aufbringen eines haftungshemmenden Chemikals auf dem fluorierten Polymerfilm od. dgl. (8) oder durch Aufheben einer Folienätzung, vorzugsweise mittels einer kurzzeitigen punktuellen Aufbringung einer Temperatur zwischen 250 und 400°C, vorzugsweise zwischen 300 und 320 °C erfolgt.

## Claims

1. A method for producing a pharmaceutical stopper, piston or similar seal (2 - 2f) which consists essentially of rubber or a similar elastomeric material and serves to close or subdivide a bottle (1), a barrel (22) of a syringe, or a similar container, the stopper, piston or the like having in a sealing area facing the interior (6) of the container a coating of a fluorinated polymerfilm (8) or similar inert film only partly surrounding said sealing area of the stopper at the side thereof facing the wall of the container opening, so that a further part of the uncoated sealing area of the stopper sealingly butts against the container opening (3), characterized in that the stopper or the like is produced in several working steps, in the first step an unvulcanized rubber sheet (46) together with a fluorinated polymer film or similar inert film (8) is initially pressed into a calibre plate (41) of a first moulding tool (45) and then corresponding portions of the polymer film or the like (8) and of the rubber sheet (46) assume the outer contour corresponding to the hollow mould(s) (47) provided in the calibre plate (41), polymer film or the like (8) resting against the wall of the hollow mould (47) being inseparably connected underthe influence of heat to the part of the rubber sheet (46) lying within and being vulcanized to form in each case one inner sealing part (46a) of the stopper (2), that in the next working step the inner sealing parts (46a) of the stopper (2) which have thus been formed and are coated with the fluorinated polymer or the like (8) are removed from the first moulding tool (45) and in the next working step are punched out of the coated rubber sheet (46) in such a manner that the upper edge (52) of each inner rubber sealing part (46a) protrudes somewhat radially with a diameter (D 2) beyond the diameter (D 1) given by the hollow mould (47), that in the next working step in which the innersealing part (46a) is placed in the hollow mould(s) (47a) of a second moulding tool (48, 48a) said protruding upper edge (52) upwardly (at 51) seals said hollow mould(s) in such a way as to prevent that in the next working step, when the inner sealing part (46a) is joined to an outer part (5) of the stopper, unvulcanized rubber material of the subsequent outer part of the stopper enters the area between fluorinated polymer film or the like (8) and the wall of the hollow mould (47a, 47b) of the second moulding tool (48, 48a).

2. The method as claimed in claim 1, characterized in that an unvulcanized rubber sheet or similar caoutchouc plate is connected to a fluorinated polymer film or the like (8), possibly with the use of adhesive agents, appropriately by rolling on.

3. The method as claimed in claim 1 or claim 2, characterized in that the fluorinated polymer film or the like (3) and the unvulcanized rubber sheet (46) or similar caoutchouc plate are interconnected before they are placed between the counter- plate (42) and the moulding plate (41) of the first moulding tool (45).

4. The method as claimed in any one of claims 1 to 3, characterized in that the closing operation of the first moulding tool (45) in the first working step is controlled in such a way that as the pressure slowly rises the unvulcanized rubber sheet (46) or similar caoutchouc plate penetrates into the hollow mould(s) (47) of the first moulding tool (45) and deep draws the fluorinated polymerfilm or the like (8) so as to be free from cracks and folds, the corresponding portions of the unvulcanized rubber sheet (46) or the like acting as a pressure pad.

5. The method as claimed in any one of claims 1 to 4, characterized in that the fluorinated polymer film (3) or the like serving to coat the stopper (2) or the like has a thickness of between 0.01 and 0.5 millimetres.

6. The method as claimed in any one of claims 1 to 5, characterized in that the inner sealing part (46a) belonging to the stopper or the like (2) and produced in the first working step initially remains still deformable and undergoes further deformation inside the second moulding tool (48), a bulge (58) being formed which preferably projects somewhat radially beyond the remaining diameter (D 1) of the sealing part.

7. The method as claimed in any one of claims 1 to 6, characterized in thatforthe outer part (5) of the stopper a different rubber material is used than the innersealing part (46a) belonging to the stopper or the like (2 - 2f) and bearing the fluorinated polymer film or the like (8).

8. The method as claimed in any one of claims 1 to 7, characterized in that the adhesion of the fluorinated polymer film or the like (8) relative to the inner sealing part (46a) orthe like is counteracted by producing an inert state in prescribed areas - e.g. the puncture point (63) - before the connection of polymer film or the like and inner sealing part or the like.

9. The method as claimed in claim 8, characterized in that the inert state is produced by punctual application of a chemical checking adhesion on the fluorinated polymer film or the like (8) or by neutralizing etching of the foil, preferably by means of a short, punctual application of a temperature between 250° and 400°C, preferably between 300° and 320°C.

## Revendications

1. Procédé de fabrication d'un bouchon pharmaceutique, piston ou pièce d'étanchement similaire (2 - 2f), consistant substantiellement en du caoutchouc ou en un élastomère similaire, en vue de l'obturation et de la subdivision d'un flacon (1), d'un cylindre (22) de seringue ou d'un réceptacle similaire, le bouchon, piston ou pièce similaire présentant, dans une zone d'étanchéité tournée vers l'espace interne (6) du réceptacle, un revêtement en un film (8) de polymère fluoré ou film inerte similaire, qui entoure seulement en partie cette zone d'étanchéité du bouchon sur son côté tourné vers la paroi de l'embouchure du réceptacle, de sorte qu'une autre partie de la zone d'étanchéité du bouchon non revêtue vient s'appliquer, de manière étanche, contre l'embouchure (3) du réceptacle, caractérisé par le fait que la fabrication du bouchon ou pièce similaire se déroule en plusieurs étapes opératoires et, lors de la première étape, une nappe de caoutchouc (46) non vulcanisée est tout d'abord bourrée, conjointement à un film de polymère fluoré ou film inerte similaire (8), dans une plaque de calibrage (41) d'un premier outil de formage (45), des tronçons correspondants du film de polymère ou film similaire (8) et de la nappe de caoutchouc (46) prenant alors la configuration extérieure correspondant au(x) moule(s) (47) en creux prévu(s) dans la plaque de calibrage (41), auquel cas le film de polymère ou film similaire (8) appliqué contre la paroi du moule (47) en creux se lie de manière indissociable, sous l'action de la chaleur, à la partie interne de la nappe de caoutchouc (46) et se vulcanise pour former une partie intérieure respective d'étanchement (46a) du bouchon (2) ; par le fait que, lors de l'étape opératoire suivante, les parties intérieures d'étanchement (46a) du bouchon (2) ainsi formées, revêtues du polymère fluoré ou substance similaire (8), sont prélevées du premier outil de formage (45), puis, lors de l'étape opératoire suivante, sont découpées à l'emporte-pièce, à partir de la nappe de caoutchouc (46) revêtue, de telle sorte que le bord supérieur (52) de chaque partie intérieure d'étanchement (46a) en caoutchouc soit sensiblement en saillie, dans le sens radial, d'une valeur diamétrale (D2) excédant le diamètre (D1) préétabli par le moule (47) en creux ; et par le fait que, lors de l'étape opératoire suivante, au cours de laquelle la partie intérieure d'étanchement (46a) est placée dans le (les) moule(s) (47a) en creux d'un second outil de formage (48, 48a), ce bord supérieur saillant (52) assure l'étanchéité dudit (desdits) moule(s) en creux, vers le haut (en 51), de manière telle que, lors de l'étape opératoire suivante, au stade de la solidarisation de la partie intérieure d'étanchement (46a) avec une partie extérieure (5) du bouchon, du caoutchouc non vulcanisé de la future partie extérieure du bouchon soit empêché de pénétrer dans la région située entre le film de polymère fluoré ou film similaire (8), et la paroi du moule (47a, 47b) en creux du second outil (48, 48a).

2. Procédé selon la revendication 1, caractérisé par le fait qu'une nappe de caoutchouc non vulcanisée ou plaque de caoutchouc similaire est reliée à un film de polymère fluoré ou film similaire (8), éventuellement avec utilisation d'agents d'adhérence, commodément par cylindrage.

3. Procédé selon la revendication 1 ou 2, caractérisé par le fait que le film de polymère fluoré ou film similaire (8) et la nappe de caoutchouc (46) non vulcanisée ou plaque de caoutchouc similaire sont reliés mutuellement avant d'être engagés entre la plaque complémentaire (42) et la plaque de moulage (41) du premier outil de formage (45).

4. Procédé selon l'une des revendications 1 à 3, caractérisé par le fait que le processus de fermeture du premier outil de formage (45) est commandé, lors de la première étape opératoire, de telle sorte que la nappe de caoutchouc (46) non vulcanisée ou plaque de caoutchouc similaire pénètre dans le (les) moule(s) (47) en creux du premier outil de formage (45), sous une pression croissant lentement, et que les tronçons correspondants de la nappe de caoutchouc (46) non vulcanisée ou élément similaire, agissant comme des tampons de pression, provoquent alors l'emboutissage profond du film de polymère fluoré ou film similaire (8), avec absence de fissures et de plis.

5. Procédé selon l'une des revendications 1 à 4, caractérisé par le fait que le film de polymère fluoré ou film similaire (8), servant au revêtement du bouchon (2) ou élément similaire, présente une épaisseur comprise entre 0,01 et 0,5 millimètre.

6. Procédé selon l'une des revendications 1 à 5, caractérisé par le fait que la partie intérieure d'étanchement (46a) du bouchon ou élément similaire (2), fabriquée lors de la première étape opératoire, demeure tout d'abord encore déformable et subit une déformation supplémentaire à l'intérieur du second outil de formage (48), avec formation d'un bourrelet (58) de préférence légèrement en saillie, dans le sens radial, au-delà du reste du diamètre (D1) de la partie d'étanchement.

7. Procédé selon l'une des revendications 1 à 6, caractérisé par le fait qu'on utilise, pour la partie extérieure (5) du bouchon, un caoutchouc autre que pour la partie intérieure d'étanchement (46a) du bouchon ou élément similaire (2 - 2f), qui porte le film de polymère fluoré ou film similaire (8).

8. Procédé selon l'une des revendications 1 à 7, caractérisé par le fait que l'adhérence du film de polymère fluoré ou film similaire (8) par rapport à la partie intérieure d'étanchement (46a) ou partie similaire est supprimée, par neutralisation dans des zones préétablies - par exemple la zone de perforation (63) -, avant la liaison entre le film de polymère ou film similaire et la partie intérieure d'étanchement ou partie similaire.

9. Procédé selon la revendication 8, caractérisé par le fait que la neutralisation a lieu par dépôt ponctuel d'une substance chimique anti-adhérente sur le film de polymère fluoré ou film similaire (8) ou bien par suppression d'une attaque corrosive de la feuille, de préférence par une brève exposition ponctuelle à une température comprise entre 250 et 400° C, de préférence entre 300 et 320° C.
